(19) Europäisches Patentamt
European Patent Office
Office européen des brevets

(11) Numéro de publication : **0 011 043**
**B2**

# (12) NOUVEAU FASCICULE DE BREVET EUROPÉEN

(45) Date de publication du nouveau fascicule du brevet : **26.03.86**

(21) Numéro de dépôt : **79420053.5**

(22) Date de dépôt : **22.10.79**

(51) Int. Cl.[4] : **C 07 C 53/08**, C 07 C 51/12, C 07 C 69/14, C 07 C 67/36

## (54) Procédé de préparation de l'acide acétique ou de ses mélanges avec l'acétate de méthyle.

(30) Priorité : **31.10.78 FR 7831635**

(43) Date de publication de la demande : **14.05.80 Bulletin 80/10**

(45) Mention de la délivrance du brevet : **02.09.81 Bulletin 81/35**

(45) Mention de la décision concernant l'opposition : **26.03.86 Bulletin 86/13**

(84) Etats contractants désignés : **AT BE CH DE FR GB IT LU NL SE**

(56) Documents cités :
**DE-A- 763 693**
**FR-A- 2 370 023**
**Chemie-Ing.-Techn. 37 (1965), S. 383-388**
**"Chemierohstoffe aus Kohle", G. Thieme Verlag 1977**
**Bull. Chem. Soc. Jpn. 37(1964), S. 236-241**
**Le dossier contient des informations techniques présentées postérieurement au dépôt de la demande et ne figurant pas dans le présent fascicule.**

(73) Titulaire : **RHONE-POULENC CHIMIE DE BASE**
**25, quai Paul Doumer**
**F-92408 Courbevoie (FR)**

(72) Inventeur : **Gauthier-Lafaye, Jean**
**21, rue Duguesclin**
**F-69006 Lyon (FR)**
Inventeur : **Perron, Robert**
**La Pecolière**
**F-69390 Charly (FR)**

(74) Mandataire : **Rioufrays, Roger et al**
**RHONE-POULENC RECHERCHES Service Brevets Chimie et Polymères Centre de Recherches de Saint-Fons B.P. 62**
**F-69192 St-Fons Cedex (FR)**

**EP 0 011 043 B2**

## Description

La présente invention a pour objet un procédé perfectionné pour la préparation de l'acide acétique ou de mélanges de l'acide acétique et de l'acétate de méthyle, par carbonylation du méthanol, en présence de cobalt et d'au moins un promoteur iodé.

Il est bien connu que l'acide acétique, éventuellement en mélange avec l'acétate de méthyle, peut être produit par réaction du méthanol et du monoxyde de carbone, dans des conditions relativement sévères de température et de pression, en présence de cobalt et d'iode libre ou combiné.

C'est ainsi qu'il a d'abord été proposé (Cf. brevet allemand 897 403) d'utiliser un système catalytique simple constitué par le bromure ou d'iodure de cobalt.

Puis de nombreux travaux ont suggéré des solutions pour améliorer l'efficacité du système envisagé. Il a, notamment, été proposé d'utiliser des complexes du cobalt obtenus par réaction des halogénures de cobalt avec des composés organiques d'ammonium ou de phosphonium, complexes de formule générale :

$$[A_4R]_2CoX_4 \qquad (I)$$

dans laquelle X représente un atome de brome ou d'iode, R un atome de phosphore ou d'azote, A étant un radical alcoyle inférieur, par exemple. (Cf. Brevet des Etats-Unis d'Amérique n° 2 727 902).

Ces complexes peuvent être engagés sous cette forme dans la réaction en cause, ou bien ils peuvent être formés in-situ. Néanmoins, bien que la pression et la température de la réaction de carbonylation soient élevées (600 à 700 atmosphères, 200 à 250 °C environ), l'efficacité du système catalytique, exprimée en terme de productivité horaire, reste très faible.

Cette productivité exprimée soit par rapport au volume réactionnel, soit par rapport à la masse de cobalt mise en œuvre, a pu être sensiblement améliorée en engageant, dans la réaction en cause, un halogénure de cobalt et un composé organique d'ammonium ou de phosphonium en quantité supérieure à celle requise par la stœchiométrie de formation des complexes de formule (I) ci-avant. (Cf. brevet allemand 933 148).

Toutefois, les conditions de température et de pression restent tout aussi sévères.

Plus récemment, il a été proposé d'adjoindre au système catalytique (cobalt, halogène) l'un des métaux du groupe constitué par le palladium, le platine, l'iridium, le ruthénium ou le cuivre. Si on a pu ainsi réaliser la réaction précitée dans des conditions sensiblement moins sévères, l'efficacité du système catalytique exprimée en terme de productivité horaire est restée médiocre. (Cf. brevet belge 808716).

Il a maintenant été trouvé qu'il est possible d'obtenir l'acide acétique ou ses mélanges avec l'acétate de méthyle par carbonylation du méthanol en présence de cobalt et d'au moins un promoteur iodé, avec une productivité accrue, même dans des conditions de température et de pression relativement peu sévères.

La présente invention a donc pour objet un procédé perfectionné de carbonylation du méthanol, en présence d'hydrogène, en présence de cobalt, qui permet notamment d'augmenter de manière appréciable la productivité horaire en dérivés acétiques (acétate de méthyle et/ou acide acétique), par rapport à la quantité de cobalt engagée dans la réaction, et cela avec un gain de productivité par rapport au volume réactionnel, même lorsque les conditions de température et de pression sont peu sévères.

Les recherches, qui ont abouti à la présente invention, ont montré d'une manière tout à fait inattendue que l'on peut obtenir sélectivement les dérivés acétiques (acétate de méthyle et/ou acide acétique) en faisant réagir sur le méthanol un mélange d'oxyde de carbone et d'hydrogène, renfermant au moins 75 % (en mole) de monoxyde de carbone, à une température inférieure ou égale à 180 °C, en présence d'une quantité efficace de cobalt, d'au moins un iodure ionique et d'au moins un halogénure covalent, le rapport des ions-grammes $I^-$ en provenance de l'iodure ionique, au nombre d'atomes-grammes de cobalt engagés étant au moins égal à 10 et le nombre d'atomes-grammes d'halogène, en provenance de l'halogénure covalent, mis en œuvre représentant de 1 à 30 % du nombre d'ions-grammes $I^-$.

Selon l'invention, il a été découvert de manière tout à fait surprenante que des résultats remarquables, exprimés notamment en terme de productivité en dérivés acétiques, peuvent être obtenus par l'utilisation conjointe des deux types de « promoteurs » mentionnés ci-avant, l'iodure ionique étant engagé en quantité telle que le rapport ($I^-/Co$) soit supérieur ou égal à 10 et cela avec une faible proportion d'halogénure covalent.

Le procédé selon l'invention nécessite la mise en œuvre d'au moins un iodure ionique. Par iodure ionique, on entend les iodures minéraux ou organiques pris isolément ou en mélange, dont les cations sont choisis parmi les cations des métaux alcalins, les cations des métaux alcalino-terreux, les cations ammonium, ammonium quaternaires, les cations phosphonium, phosphonium quaternaires. La nature précise du cation ammonium ou phosphonium n'est pas fondamentale, dans le cadre de la présente invention.

Le choix parmi ces composés est davantage orienté par des considérations d'ordre pratique, telles que la solubilité, la disponibilité et la commodité d'emploi. A ce titre, les iodures d'ammonium

quaternaires dérivés des trialcoylamines inférieures, telles que la triméthylamine, la triéthylamine, tributylamine, ou bien correspondant soit à des alcoyl-arylamines telles que la diméthylaniline, soit à des amines tertiaires cycliques telles que les N-alcoyl-pyrrolidines ou pipéridines, les N-alcoyl-hexaméthylène imines, le pyrrole, la pyridine, les alcoylpyridines, quaternisés par traitement avec des esters de l'acide iodhydrique et d'alcools aliphatiques conviennent particulièrement bien. Les iodures d'ammonium bis-quaternaires, dérivés par exemple des tétraalcoylalcoylène diamines peuvent également être utilisés dans le cadre de la présente invention.

L'utilisation des iodures de phosphonium dérivés par exemple des triarylphosphines, d'accès facile, telles que la triphénylphosphine, la tritolylphosphine, la trixylylphosphine ou des trialkylphosphines d'accès facile telles que la triméthylphosphine, la tributylphosphine ou la tricyclohexylphosphine et quaternisés par des iodures d'alcoyles ou d'aralcoyles, est également envisagée dans le cadre du présent procédé. Les iodures de phosphonium bis-quaternaires, dérivés par exemple des bis-$\alpha$, $\omega$ (diphénylphosphino) alcanes peuvent également être utilisés dans le cadre de la présente invention.

Un mode préféré de réalisation de la présente invention comprend l'utilisation d'iodures des métaux alcalins ou alcalino-terreux, tels que : LiI, NaI, KI, CsI et $CaI_2$. De préférence on utilise un ou plusieurs iodures des métaux alcalins et de manière encore plus avantageuse NaI ou KI.

Selon la présente invention, le rapport ($I^-$/Co), $I^-$ provenant de l'iodure ionique, doit être au moins égal à 10. Bien que ce rapport puisse varier dans de larges limites, il ne semble pas utile de dépasser une valeur de l'ordre de 200. Selon un mode de réalisation particulièrement avantageux du procédé selon l'invention, le rapport $I^-$/Co est supérieur ou égal à 20.

Le procédé selon l'invention exige également la mise en œuvre d'au moins un halogénure covalent. Par halogénure covalent, on entend d'une part les chlorures, les bromures et, de préférence, les iodures d'hydrocarbyles, c'est-à-dire, des composés formés par remplacement, au moyen d'halogène, d'au moins un atome d'hydrogène d'une molécule organique ne contenant que du carbone et de l'hydrogène, et, d'autre part, les composés qui dans les conditions de réaction sont susceptibles de conduire à l'apparition d'un halogénure de méthyle dans le milieu réactionnel, choisis parmi $Cl_2$, $Br_2$, $I_2$, HCl, HBr, HI, $CoBr_2$ et $CoI_2$.

L'invention envisage notamment l'utilisation des chlorures, bromures et iodures d'alcoyles inférieurs ayant de 1 à 4 atomes de carbone dans la molécule, tels que le bromure et l'iodure de méthyle, le bromure et l'iodure d'éthyle.

De préférence, on utilise l'iodure de méthyle ou l'une de ses sources potentielles choisies parmi l'iode, l'acide iodhydrique et l'iodure de cobalt.

Selon la présente invention, l'effet apporté par halogénure covalent est appréciable même pour de faibles teneurs, de l'ordre de I atome-gramme d'halogène (désigné par X dans ce qui suit) pour 100 ions-grammes d'iodure, c'est-à-dire pour un rapport

$$X/I^-$$

de l'ordre de 1 %.

Il n'est pas souhaitable de dépasser la valeur de 30 % pour ce rapport, notamment pour des raisons technologiques, en particulier pour limiter la corrosion de l'appareillage.

On opère de préférence avec un rapport $X/I^-$ compris entre 3 et 25 %.

Le procédé selon l'invention est réalisé en présence de cobalt. N'importe quelle source de cobalt qui réagira avec l'oxyde de carbone dans le milieu de réaction pour donner un complexe cobalt-carbonyle, hydro-cobalt-carbonyle ou cobalt-carbonylate peut être utilisé dans le cadre de la présente invention.

Des sources typiques de cobalt sont par exemple le cobalt métallique finement divisé, des sels inorganiques tel que le carbonate de cobalt, des sels organiques, en particulier d'un acide gras. Peuvent également être employés les cobalt-carbonyles, hydrocarbonyles, ou leurs complexes. Parmi les dérivés du cobalt convenant pour la mise en œuvre du procédé selon l'invention, on peut citer l'acétate et le formiate de cobalt, les halogénures de cobalt, en particulier l'iodure de cobalt, le dicobaltoctacarbonyle.

On opère avec une quantité efficace de cobalt. En général, cette quantité est telle que le rapport du nombre de milli atomes-grammes de cobalt au nombre de moles de méthanol ($Co/CH_3OH$) soit compris entre 0,05 et 1.

Bien entendu lorsque la source de cobalt est un halogénure il n'est pas indispensable d'introduire un halogénure covalent, tel que $X_2$ ou $CH_3X$, X représentant un atome de chlore, de brome ou d'iode. Toutefois l'addition d'un tel composé peut s'avérer avantageuse.

Le procédé de carbonylation selon la présente invention est conduit de préférence en phase liquide. Comme on opère le plus souvent avec du méthanol en excès, l'emploi simultané d'un solvant supplémentaire est généralement superflu mais on peut en principe faire usage de tels solvants, par exemple d'hydrocarbures, d'esters, d'éthers et des produits de la réaction.

Dans le cadre du présent procédé, il n'est pas nécessaire de purifier ou de déshydrater le méthanol au préalable. On peut utiliser le méthanol de qualité technique. Le méthanol utilisé au départ peut contenir jusqu'à 50 % d'eau et/ou d'acétate de méthyle.

De même, on peut utiliser dans le cadre de la présente invention les halogénures covalents, les iodures ioniques et les composés à base de cobalt, du commerce.

Selon le présent procédé, on fait réagir sur le méthanol un mélange de monoxyde de carbone et d'hydrogène. Il est essentiel que ledit mélange renferme au moins 75 % en mole de monoxyde de carbone. On utilise de préférence des mélanges renfermant de 80 à 98 % de monoxyde de carbone. Le mélange de gaz peut renfermer des impuretés telles que, par exemple, du dioxyde de carbone, de l'oxygène, du méthane, et de l'azote.

La réaction nécessite la mise en œuvre d'une pression totale, généralement comprise entre 50 et 600 bars. De préférence, la pression totale est comprise entre 75 et 350 bars, et plus particulièrement entre 100 et 320 bars.

La température de réaction est d'au plus 180 °C environ et peut être aussi faible que 100 °C. De préférence, la température de réaction se situe dans la gamme allant de 120 à 175 °C.

Les exemples ci-après illustrent la mise en œuvre du procédé selon l'invention sans toutefois en limiter le domaine ou l'esprit.

Les conventions utilisées dans les exemples ci-après sont les suivantes :

AcOMe désigne l'acétate de méthyle

AcOH désigne l'acide acétique

$C_4$ désigne l'ensemble des produits formés, ayant 4 atomes de carbone dans la molécule, à savoir le buténal et/ou le butanal et/ou le butanol.

MeOMe désigne le diméthyléther (produit recyclable dans la réaction). RR pour un produit donné, est égal au rapport du nombre de moles du produit considéré, formées pendant la réaction, au nombre de moles de méthanol engagées à la réaction, à l'exception de RR ($C_4$) et de RR (MeOMe) qui sont égaux au double du rapport défini ci-avant. ΣRR est la somme des RR, pour l'ensemble des produits liquides observés à l'exception du diméthyl-éther.

Les productivités horaires sont exprimées en grammes d'acide acétique potentiel par heure, rapportés soit au volume réactionnel (g/h × 1) soit à la masse de cobalt engagée (g/h × g.Co).

## Exemples 1 à 5

Une première série d'essais a été réalisée selon le mode opératoire décrit ci-après pour l'Exemple 1.

Dans un autoclave en acier inoxydable Z 8—CNDT 17 — 12 (norme AFNOR) de 125 ml de capacité, on charge :

100 ml de méthanol
0,58 milli atome-gramme de cobalt sous la forme de dicobaltoctacarbonyle
24 millimoles d'iodure de sodium
1 millimole d'iodure d'éthyle
($Co/CH_3OH = 0,237$)

Après fermeture de l'autoclave, on établit une pression de 140 bars à l'aide d'un mélange $CO/H_2$ : 95/5 (pression initiale d'hydrogène = 7 bars). L'agitation par un système de va et vient est mise en route et l'autoclave est porté à 160 °C, en 20 mm environ, au moyen d'un four annulaire. La pression totale dans l'autoclave est alors de 250 bars et elle est maintenue entre 238 et 250 bars par des recharges périodiques de monoxyde de carbone. Après 1 h 40 mn de réaction à la température indiquée, (absorption totale de 104 bars de monoxyde de carbone) le chauffage et l'agitation sont arrêtés ; l'autoclave est refroidi et dégazé.

Le mélange réactionnel résultant est analysé par chromatographie gazeuse, après avoir été dilué à l'eau et acidifié par de l'acide sulfurique 36 N.

Les conditions particulières, ainsi que les résultats obtenus sont portés dans le Tableau (I) ci-après :

(Voir tableau I page 5)

Tableau I

| EXEMPLE N° | IODURE IONIQUE | | HALOGENURE COVALENT | | RR / ΣRR en % | | PRODUCTIVITE | |
|---|---|---|---|---|---|---|---|---|
| | NATURE | $I^-/Co$ | NATURE | $X/I^-$ en % | AcOH + AcOMe | AcOH | g/h / l | g/h × gCo |
| 1 | NaI | 41 | $C_2H_5I$ | 4.2 | 91 | 10,5 | 140 | 410 |
| 2(**) | K I | 21 | $CH_3I$ | 25.0 | 75 | 8.4 | 130 | 370 |
| 3 | $[(CH_3)(C_6H_5)_3P]I$ | 31 | $CH_3I$ | 4.4 | 90 | 7.0 | 150 | 430 |
| 4(***) | $[(C_2H_5)_4N]I$ | 33 | $C_2H_5Br$ | 12.1 | 90 | 14.4 | 130 | 370 |
| 5(*) | $NH_4I$ | 52 | $CH_3I$ | 10.0 | 97 | 10.3 | 70 | 210 |

(*) Co $CO_3$ ; durée de réaction 2 heures 55 minutes.
(**) Mélange initial $CO/H_2$ = 80 : 20 (pression initiale d'hydrogène : 28 bars)
(***) T = 180 °C.
N.B. Dans l'ensemble de ces essais on n'observe pas de $C_4$

Exemples 6 à 13

On a réalisé une série d'essais selon le mode opératoire décrit précédemment. On a chargé, sauf indications contraires :

100 ml de méthanol
0,58 milli atome-gramme de cobalt sous la forme de dicobaltoctacarbonyle
12 millimoles d'iodure de potassium
3 millimoles d'iodure de méthyle

On a donc :

$Co/CH_3OH = 0,237$
$I^-/Co = 21$
$X/I^- = 25\ \%$

La durée de réaction, sauf indication contraire est de 1 h 40 mn. Les conditions particulières ainsi que les résultats obtenus sont portés dans le Tableau II ci-après, dans lequel $P(H_2)$ désigne la pression initiale d'hydrogène, et $P_T$, la pression totale.

(Voir tableau II page 7)

Tableau II

| EXEMPLE N° | PRESSION EN BARS | | TEMPERATURE EN °C | RR / ΣRR (en %) | | PRODUCTIVITE | |
|---|---|---|---|---|---|---|---|
| | P ($H_2$) | $P_T$ | | AcOH | AcOH + AcOMe | g/h × 1 | g/h × g-Co |
| 6 | 28 | 250 | 140 | 8.1 | 74 | 91 | 260 |
| 7 | 7 | 250 | 120 | 8.3 | 91 | 49 | 120 |
| 8 | 7 | 250 | 140 | 14.1 | 88 | 90 | 260 |
| 9 | 7 (*) | 250 | 140 | 40.5 | 87 | 78 | 230 |
| 10 | 28 | 200 | 160 | 8.2 | 68 | 96 | 280 |
| 11 (**) | 28 | 250 | 160 | 13.3 | 79 | 160 | 460 |
| 12 (***) | 28 | 250 | 160 | 24 3 | 66 | 97 | 280 |
| 13 (****) | 28 | 250 | 160 | 15.7 | 70 | 90 | 280 |

(*) Cette pression d'hydrogène est maintenue tout au long de la réaction.
(**) La charge est composée de 90 ml de méthanol at 10 ml de N-méthylpyrrolidone ; ($Co/CH_3OH$ = 0,213)
(***) La charge est composée de 70 ml de méthanol et de 30 ml d'acétate de méthyle ; ($Co/CH_3OH$ = 0,166)
(***) La charge est composée de 90 ml de méthanol et de 20 ml d'eau ; ($Co/CH_3OH$ = 0,213 ; durée de réaction : 2h.)

**Revendications**

1. Procédé de préparation de dérivés acétiques par réaction sur le méthanol d'un mélange comprenant de l'hydrogène et du monoxyde de carbone, le monoxyde de carbone représentant au moins 75 % en mole, à une température inférieure ou égale à 180 °C, en présence d'une quantité efficace de cobalt, caractérisé en ce que l'on opère en présence initiale d'au moins un iodure ionique et d'au moins un halogénure covalent, le cation de l'iodure ionique étant choisi dans le groupe constitué par les cations des métaux alcalins, les cations des métaux alcalino-terreux, les cations ammonium, les cations ammonium quaternaires, les cations phosphonium et les cations phosphonium quaternaires, l'halogénure covalent étant choisi parmi les chlorures, bromures et iodures d'hydrocarbyles, le chlore, le brome et l'iode moléculaires, les acides halohydriques correspondants, le bromure et l'iodure de cobalt, le rapport $I^-/Co$ (en Atomes-grammes) étant supérieur ou égal à 10, et le rapport $X/I^-$ (en Atomes-grammes) dans lequel X représente un atome d'halogène en provenance d'un halogénure covalent, étant compris entre 1 et 30 %.

2. Procédé selon la revendication 1, caractérisé en ce qu'on opère en phase liquide.

3. Procédé selon la revendication 1, caractérisé en ce que $X/I^-$ est compris entre 3 et 25 %.

4. Procédé selon la revendication 1 ou 3, caractérisé en ce que l'iodure ionique est choisi dans le groupe constitué par les iodures alcalins et les iodures alcalino-terreux.

5. Procédé selon la revendication 4, caractérisé en ce que l'iodure ionique est choisi dans le groupe constitué par LiI, NaI, KI, CsI et $CaI_2$.

6. Procédé selon la revendication 1 ou 5, caractérisé en ce que $I^-/Co$ est supérieur ou égal à 20.

7. Procédé selon la revendication 1 ou 3, caractérisé en ce que l'halogénure covalent est choisi dans le groupe constitué par les chlorures, les bromures et les iodures d'alcoyles ayant de 1 à 4 atomes de carbone dans la molécule, le chlore, le brome et l'iode moléculaires, les acides halohydriques correspondants, le bromure et l'iodure de cobalt.

8. Procédé selon la revendication 7, caractérisé en ce que l'halogénure covalent est choisi parmi l'iodure de méthyle, l'iode moléculaire et l'acide iodhydrique.

9. Procédé selon la revendication 1, caractérisé en ce que la quantité de cobalt présente dans le milieu est comprise entre 0,05 et 1 milli atome-gramme par mole de méthanol.

10. Procédé selon la revendication 1, caractérisé en ce que la température de la réaction est comprise entre 120 et 175 °C.

11. Procédé selon la revendication 1, caractérisé en ce que la pression totale est comprise entre 50 et 600 bars, et de préférence entre 75 et 350 bars.

12. Procédé de préparation de dérivés acétiques par réaction en phase liquide, sur le méthanol, d'un mélange comprenant de l'hydrogène et du monoxyde de carbone, le monoxyde de carbone représentant au moins 75 % en mole, à une température comprise entre 120 et 175 °C, sous une pression totale comprise entre 75 et 350 bars, en présence de 0,05 à 1 milli atome-gramme de cobalt par mole de méthanol, en présence d'un iodure alcalin, $I^-/Co$ étant compris entre 20 et 200, et en présence initiale d'iodure de méthyle, $CH_3I/I^-$ étant compris entre 3 et 25 % (molaire).

13. Procédé selon la revendication 4 ou 12, caractérisé en ce que l'iodure ionique est choisi dans le groupe constitué par NaI et KI.

14. Procédé selon la revendication 11 ou 12, caractérisé en ce que la pression totale est comprise entre 100 et 320 bars.

**Claims**

1. Process for the preparation or acetic derivatives by reacting, with methanol, a mixture comprising hydrogen and carbon monoxide, the carbon monoxide reprenting a least 75 mol %, at a temperature lower than or egal to 180 °C, in the presence of an effective amount of cobalt, characterised in that the process is carried out in the initial presence of at least one ionic iodide and at least one covalent halide, the cation of the ionic iodide being chosen from the group consisting of alkali metal cations, alkaline earth metal cations, ammonium cations, quaternary ammonium cations, phosphonium cations and quaternary phosphonium cations, the covalent halide being selected from the group consisting of the chlorides, bromides and the iodides of hydrocarbyles, molecular chlorine, bromine and iodine and the corresponding halohydric acids, cobalt bromide and iodide, the ratio $I^-/Co$ (in gram-atoms) being greater than or equal to 10, and the ratio $X/I^-$ (in gram-atoms), in which X represents a halogen atom originating from a covalent halide, being between 0,01 and 0,3.

2. Process according to claim 1, characterised in that it is carried out in the liquid phase.

3. Process according to claim 1, characterised in that $X/I^-$ is between 0.03 and 0.25.

4. Process according to claims 1 or 3, characterised in that the ionic iodide is chosen from the group consisting of alkali metal iodides or alkaline earth metal iodides.

5. Process according to claim 4, characterised in that the ionic iodide is chosen from the group consisting of LiI, NaI, KI, CsI, and $CaI_2$.

6. Process according to claims 1 or 5, characterised in that $I^-/Co$ is greated than or equal to 20.

7. Process according to claims 1 or 3, characterised in that the covalent halide is chosen from the

group consisting of the chlorides, the bromides and the iodides of alkyls having from 1 to 4 carbon atoms in the molecule, molecular chlorine, bromine and iodine, the corresponding hydrogen halide acids, cobalt bromide and iodide.

8. Process according to claim 7, characterised in that the covalent halide is chosen from the group consisting of methyl iodide, molecular iodine and hydroiodic acid.

9. Process according to claim 1, characterised in that the amount of cobalt present in the medium is between 0.05 and 1 milligram-atom per mol of methanol.

10. Process according to claim 1, characterised in that the reaction temperature is between 120 and 175 °C.

11. Process according to claim 1, characterised in that the total pressure is between 50 and 600 bars and preferably between 75 and 350 bars.

12. Process for the preparation of acetic derivatives by reacting, with methanol, in the liquid phase, a mixture comprising hydrogen and carbon monoxide, the carbon monoxide representing at least 75 mol %, at a temperature of between 120 and 175 °C, under a total pressure of between 75 and 350 bars, in the presence of 0.5 to 1 milligram-atom of cobalt per mol of methanol, in the presence of an alkali metal iodide, $I^-$/Co being between 20 and 200, and in the initial presence of methyl iodide, $CH_3I/I^-$ being between 0.03 and 0.25 (in mols).

13. Process according to claims 4 or 12, characterised in that the ionic iodide is chosen from the group comprising NaI and KI.

14. Process according to claims 11 or 12, characterised in that the total pressure is between 100 and 320 bars.

**Patentansprüche**

1. Verfahren zur Herstellung von Essigsäure und Essigsäureverbindungen durch Umsetzung von Methanol mit einem Gemisch, enthaltend Wasserstoff und Kohlenmonoxid, wobei das Kohlenmoxid mindestens 75 Mol-% ausmacht, bei einer Temperatur von 180 °C oder darunter, in Gegenwart einer Wirksamen Menge Kobalt, dadurch gekennzeichnet, daß man in ursprünglicher Gegenwart mindestens eines ionischen Jodids und mindestens eines kovalenten Halogenids arbeitet, wobei das Kation des ionischen Jodids ausgewählt ist aus der Gruppe, bestehend aus den Kationen der Alkalimetalle, der Erdalkalimetalle, der Ammoniumkationen, der quaternären Ammoniumkationen, der Phosphoniumkationen und der quaternären Phosphoniumkationen, wobei das kovalente Halogenid ausgewählt ist aus der Gruppe bestehend aus den Kohlenwasserstoffchloriden, -bromiden und -jodiden, molekularem Chlor, Brom and Jod, den entsprechenden Halogenwasserstoffsaüren, Kobaltbromid und Kobaltjodid, das Verhältnis $J^-$/Co (in gAtom) gleich oder größer 10 is und das Verhältnis $X/J^-$ (in gAtom) 1 bis 30 % ausmacht, wobei X ein Halogenatom bedeutet, das aus einem Kovalenten Halogenid stammt.

2. Verfahren nach Anspruch 1, dadurch gekennzeichnet, daß man in flüssiger Phase arbeitet.

3. Verfahren nach Anspruch 1, dadurch gekennzeichnet, daß $X/J^-$ 3 bis 25 % beträgt.

4. Verfahren nach Anspruch 1 oder 3, dadurch gekennzeichnet, daß das ionische Jodid ausgewählt wird aus der Gruppe, bestehend aus den Alkali- oder Erdalkalijodiden.

5. Verfahren nach Anspruch 4, dadurch gekennzeichnet, daß das ionische Jodid ausgewählt wird aus der Gruppe, bestehend aus LiJ, NAJ, KJ, CsJ und $CaJ_2$.

6. Verfahren nach Anspruch 1 oder 5, dadurch gekennzeichnet, daß $J^-$/Co größer als oder gleich 20 ist.

7. Verfahren nach Anspruch 1 oder 3, dadurch gekennzeichnet, daß das kovalente Halogenid ausgewählt wird aus der Gruppe bestehend aus den Alkylchloriden, Alkylbromiden und Alkyljodiden mit jeweils 1 bis 4 Kohlenstoffatomen im Molekül, molekularem Chlor, Brom oder Jod, oder den entsprechenden Halogenwasserstoffsäuren, Kobaltbromid oder Kobaltjodid.

8. Verfahren nach Anspruch 7, dadurch gekennzeichnet, daß das kovalente Halogenid ausgewählt wird aus der Gruppe bestehend aus Methyljodid, molekularem Jod und Jodwasserstoffsäure.

9. Verfahren nach Anspruch 1, dadurch gekennzeichnet, daß die in Medium vorhandene Menge Kobalt 0,05 bis 1 mgAtom je Mol Methanol ausmacht.

10. Verfahren nach Anspruch 1, dadurch gekennzeichnet, daß die Reaktionstemperatur 120 bis 175 °C beträgt.

11. Verfahren nach Anspruch 1, dadurch gekennzeichnet, daß der Gesamtdruck 50 bis 600 bar, vorzugsweise 75 bis 350 bar beträgt.

12. Verfahren zur Herstellung von Essigsäure und Essigsäureverbindungen durch Umsetzung in flüssiger Phase von Methanol mit einem Gemisch, das Wasserstoff und Kohlenmonoxid enthält, wobei das Kohlenmonoxid mindestens 75 Mol-% ausmacht, bei einer Temperatur von 120 bis 175 °C, unter einem Gesamtdruck von 75 bis 350 bar, in Gegenwart von 0,05 bis 1 mg Atom Kobalt je Mol Methanol, in Gegenwart eines Alkalijodids, wobei $J^-$/Co 20 bis 200 beträgt, und in ursprünglicher Gegenwart von Methyljodid, wobei $CH_3J/J^-$3 bis 25 Mol-% ausmacht.

13. Verfahren nach Anspruch 4 oder 12, dadurch gekennzeichnet, daß das ionische Jodid aus der Gruppe, bestehend aus NaJ und KJ ausgewählt wird.

14. Verfahren nach Anspruch 11 oder 12, dadurch gekennzeichnet, daß der Gesamtdruck 100 bis 320 bar beträgt.